# FASCICULE DE BREVET EUROPEEN

(11) **EP 1 299 054 B1**
(45) Date de publication et mention de la délivrance du brevet: **30.05.2007**
(21) Numéro de dépôt: 01984139.4
(22) Date de dépôt: 06.07.2001
(51) Int. Cl.: A61F 2/44

(54) **IMPLANT INTERSOMATIQUE**
WIRBELKÄFIGIMPLANTAT
INTERSOMATIC IMPLANT

(30) Priorité: 12.07.2000 FR 0009091
(43) Date de publication de la demande: 09.04.2003
(73) Titulaire: Abbott Spine, La Cité Mondiale, 33000 Bordeaux (FR)
(72) Inventeur: PASQUET, Denis, F-33600 Pessac (FR); LE COUEDIC, Régis, F-33000 Bordeaux (FR)
(74) Mandataire: Dronne, Guy
(86) Numéro de dépôt international: PCT/FR2001/002178
(87) Numéro de publication internationale: WO 2002/003895

(56) Documents cités:
- WO-A-99/66867
- FR-A- 2 703 580
- FR-A- 2 742 653
- US-A- 5 192 327

## Description

La présente invention concerne un implant intersomatique destiné notamment à immobiliser un greffon osseux entre deux vertèbres, et plus particulièrement une cage apte à contenir les greffons.

Les domaines d'application de l'invention sont notamment les traitements chirurgicaux des disques dégénératifs.

Ces traitements comportent généralement une intervention visant à bloquer ensemble deux vertèbres contiguës, et ce en interposant entre elles des greffons osseux qui, après que la greffe a pris, les rendent solidaires. Cependant, compte tenu des contraintes mécaniques exercées sur les greffons par les deux vertèbres, ces derniers risquent notamment d'être expulsés ou de s'effondrer.

Pour pallier cet inconvénient il est connu d'immobiliser les greffons au moyen de cages dont les extrémités supérieures et inférieures, contre les bords desquelles s'appuient les vertèbres, sont ouvertes de façon à permettre le contact des greffons avec le plateau vertébral supérieur et le plateau vertébral inférieur.

Le blocage de deux vertèbres ensemble par un élément osseux présente l'avantage de conférer des propriétés mécaniques homogènes à l'ensemble ainsi obtenu, et en particulier la déformation élastique dudit ensemble qui est caractérisée par son module d'élasticité.

Or, les cages généralement utilisées sont en alliage de titane et le module d'élasticité de ces alliages est au moins dix fois supérieur au module d'élasticité de l'os des vertèbres. Ainsi, les cages intersomatiques, restant à demeure entre les vertèbres créent-elles une discontinuité des propriétés mécaniques dans l'ensemble formé par les deux vertèbres soudées.

Afin de conserver une déformation élastique homogène on a proposé d'utiliser des cages en matériaux macromoléculaires dont les modules d'élasticité sont de l'ordre de ceux de l'os. Cependant, les cages, de forme générale annulaire, en matières plastiques, sont plus fragiles que les cages en alliage de titane et leur introduction à force entre les vertèbres peut provoquer la rupture de leur bord. La solution qui consiste à élargir leur bord diminue le volume disponible pour les greffons et donc la force de liaison entre les deux vertèbres. En outre, leur dureté étant inférieure à celles des cages en alliage de titane, leur bord pénètrent de façon moins importante dans les plateaux vertébraux, de sorte que leur immobilisation par rapport aux vertèbres est moins bien assurée.

Le brevet US-A-5,192,327 décrit un implant intersomatique conforme au préambule de la revendication 1.

Le brevet FR-A-2 742 653 décrit une cage intersomatique dans laquelle deux parois internes ont des bords inférieur et supérieur en forme d'aileron pour d'ancrer dans l'os spongieux.

Un objet de la présente invention est de fournir un implant intersomatique en matériaux macromoléculaires de type cage présentant une ouverture supérieure et une ouverture inférieure du même ordre de grandeur que celles des cages en alliage de titane de façon à conserver une surface de greffe sensiblement égale et dont le maintient en position fixe par rapport aux vertèbres est amélioré.

A cet effet, la présente invention propose un implant intersomatique caractérisé en ce qu'au moins une portion desdits seconds bords inférieur et supérieur font saillie en dehors de l'espace défini par lesdits premiers bords inférieur et supérieur de ladite première partie et sont de forme curviligne et leurs extrémités rejoignent respectivement lesdits premiers bords inférieur et supérieur de ladite première partie de telle manière que les deuxièmes bords respectent la forme biconcave de l'espace intersomatique et constituent des points d'appui supplémentaires de l'implant contre les plateaux vertébraux dans leur partie centrale.

Ainsi, une caractéristique de l'implant intersomatique réside dans le renforcement de ladite première partie de forme générale annulaire par une seconde partie allongée située dans la direction prédéterminée d'enfoncement dudit implant entre les vertèbres. De la sorte, malgré les contraintes exercées sur la première partie lors de son impaction entre les vertèbres, et qui tendent à la déformer selon son plan moyen de façon à rapprocher les deux portions sensiblement opposées, ladite seconde partie conserve sa forme générale annulaire. En outre, les surfaces d'ouverture supérieure et inférieure, divisées en deux par ladite seconde partie sont du même ordre de grandeur que les surfaces d'un implant intersomatique en alliage de titane ce qui permet d'obtenir une surface de greffe sensiblement identique.

De plus les seconds bords inférieur et supérieur font saillie en dehors de l'espace défini par lesdits premiers bords inférieur et supérieur de ladite première partie, de sorte que, non seulement l'implant est maintenu entre les vertèbres par contact des bords de ladite première partie contre les plateaux vertébraux, mais il est également maintenu par ladite deuxième partie de forme allongée qui prend appui contre les plateaux vertébraux de forme biconcave.

En outre, lesdits seconds bords inférieur et supérieur de ladite seconde partie sont de forme curviligne et leurs extrémités rejoignent respectivement lesdits premiers bords inférieur et supérieur de ladite première partie. Ainsi, la forme de la seconde partie coïncide sensiblement avec la forme de l'espace disponible situé entre les vertèbres.

Selon un premier mode de réalisation de l'invention, ladite première partie et ladite seconde partie sont reliées ensemble de façon à former une seule et même pièce, de sorte qu'elles sont parfaitement solidaires.

Selon un mode préférentiel de mise en oeuvre de l'invention ladite première partie est percée latéralement selon ledit axe principal de ladite seconde partie de façon à ménager un trou borgne se prolongeant dans ladite seconde partie, ledit trou borgne étant apte à recevoir un outil d'insertion.

Comme on l'expliquera plus en détails, selon ce mode préféré de mise en oeuvre, l'insertion de l'implant se fait à force entre deux vertèbres adjacentes et il est nécessaire de maîtriser parfaitement la direction de l'insertion. Afin de conserver un angle d'impact constant, l'outil que l'on applique contre ledit implant est introduit dans un trou borgne qui se prolonge dans la seconde partie. Ainsi, l'extrémité de l'outil appliquée contre l'implant est prisonnière dudit implant durant l'impact et ne risque pas de perforer un tissu situé dans l'environnement de l'intervention.

De façon avantageuse lesdits bords inférieurs et supérieurs desdites première et seconde parties présentent des cannelures aptes à constituer des points d'ancrage dans lesdites vertèbres.

Ainsi, la portion aiguë des cannelures pénètre dans la paroi des plateaux vertébraux. De la sorte, l'implant est maintenu entre les vertèbres sans possibilité de jeu latéral, ce qui est favorable à la prise des greffons osseux.

De manière préférentielle ladite première partie de forme générale annulaire présente au moins un axe de symétrie et avantageusement ladite seconde partie relie lesdites deux portions opposées de ladite première partie selon ledit axe de symétrie.

Cette configuration permet d'adapter parfaitement l'implant à la forme des vertèbres et notamment des corps vertébraux. Ladite seconde partie étant disposée selon l'axe de symétrie, les contraintes exercées sur l'implant lors de l'impaction sont uniformément réparties.

Selon un autre mode préférentiel de mise en oeuvre de l'invention, ladite seconde partie relie lesdites deux portions opposées de ladite première partie selon une direction faisant un angle compris entre 0 et 90° avec ledit axe de symétrie.

Ainsi, l'insertion de l'implant entre les vertèbres peut se faire de manière plus aisée lorsque le niveau auquel elle est réalisée ne permet pas d'effectuer l'intervention symétriquement par rapport à la colonne vertébrale. En effet, il est toujours nécessaire d'enfoncer l'implant selon l'axe de ladite seconde partie pour ne pas rompre ladite première partie, et certains niveaux vertébraux ne sont accessibles que latéralement. Dans ce cas, ladite seconde partie est disposée de façon oblique par rapport à l'axe de symétrie dudit implant.

Préférentiellement, ladite première partie présente une forme générale sensiblement hémicirculaire.

Selon encore un autre mode préféré de mise en oeuvre de l'invention ladite pièce est moulée dans un matériau macromoléculaire. Ainsi, elle est produite à des conditions économiques avantageuses. Préférentiellement ladite pièce est en polyéther-éther-cétone, qui est facilement moulable et présente des propriétés élastiques proches de celles de l'os.

Selon un second mode de réalisation de l'invention, ladite seconde partie comprend en outre des moyens d'ancrage débouchant respectivement dans ledit second bord supérieur et dans ledit second bord inférieur. Ainsi, lesdits moyens d'ancrage, pénètrent à force dans la surface des plateaux vertébraux, lors de l'introduction de l'implant entre les vertèbres, de sorte que ledit implant est complètement immobilisé en translation par rapport aux vertèbres et qu'il ne peut glisser par rapport à elles.

Selon un mode particulier de mise en oeuvre, lesdits moyens d'ancrage sont constitués d'une pièce d'ancrage présentant deux extrémités et en ce que ladite seconde partie présente un évidement débouchant dans lesdits seconds bord inférieur et supérieur de façon que ladite pièce d'ancrage, insérée dans ledit évidement, traverse ladite seconde partie de part en part, la première extrémité débouchant dans ledit second bord supérieur et la seconde extrémité débouchant dans ledit second bord inférieur de ladite seconde partie.

De la sorte, la pièce d'ancrage est susceptible d'être introduite dans la seconde partie, au moment de sont insertion entre les vertèbres, si elle est absolument nécessaire. En outre, les moyens d'ancrage étant réalisés d'une seule pièce, leur rigidité est accrue, et ils ne se déforment pas lors de l'insertion.

Avantageusement, ladite pièce d'ancrage est sensiblement de forme trapézoïdale, ses deux extrémités étant définies par les deux côtés non consécutifs et non parallèles entre eux. De façon préférentielle, elle présente un plan moyen, et elle est insérée dans ladite seconde partie de façon que ledit axe principal A de ladite seconde partie coupe les deux bases du trapèze et que son plan moyen soit sensiblement perpendiculaire au plan moyen de ladite première partie. Ainsi, grâce à l'évidement qui est sensiblement de forme parallélépipédique rectangle, les parois de la pièce d'ancrage coïncident avec les parois de l'évidement.

Préférentiellement, l'une des bases du trapèze forme deux angles aigus avec les deux autres côtés non parallèles entre eux, et en ce que ladite pièce d'ancrage est insérée dans ladite seconde partie de façon que la direction d'insertion dudit implant s'effectue de la plus grande base vers la plus petite base du trapèze. De la sorte, la pièce d'ancrage présente un point saillant à chaque extrémité, défini par l'angle aigu, susceptible de s'incruster plus profondément dans le corps vertébral et d'en être plus solidaire encore. En outre, la pièce d'ancrage, forme une portion de flèche, quand elle est encastrée entre les vertèbres, lorsque l'implant est enfoncé de la plus grande base du trapèze vers la plus petite. De la sorte, le déplacement de ladite pièce d'ancrage dans la direction opposée est rendu impossible par l'incrustation des points saillants dans les plateaux vertébraux. De façon préférentielle, la pièce d'ancrage est réalisée en alliage de titane et ainsi, sa rigidité permet un meilleur ancrage.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue schématique partielle latérale montrant la position de l'implant intersomatique, selon l'invention, entre deux vertèbres contiguës ;
- la Figure 2 est une vue schématique en perspective montrant l'implant intersomatique selon un premier mode de réalisation et partiellement, la portion de l'outil d'insertion appliquée contre l'implant;
- la Figure 3 est une vue schématique en perspective montrant l'implant intersomatique selon un autre mode de mise en oeuvre ;
- la Figure 4 est une vue schématique en perspective montrant l'implant intersomatique selon encore un autre mode de réalisation ;
- la Figure 5 est une vue schématique en perspective montrant un détail de l'implant intersomatique représenté sur la Figure 4 ;
- la Figure 6 est une vue schématique de face de l'implant intersomatique illustré sur la Figure 4 ;
- la Figure 7 est une vue schématique en coupe d'une pince à implant intersomatique ; et,
- la Figure 8 est une schématique de dessus de la pince à implant intersomatique représentée sur la Figure 7.

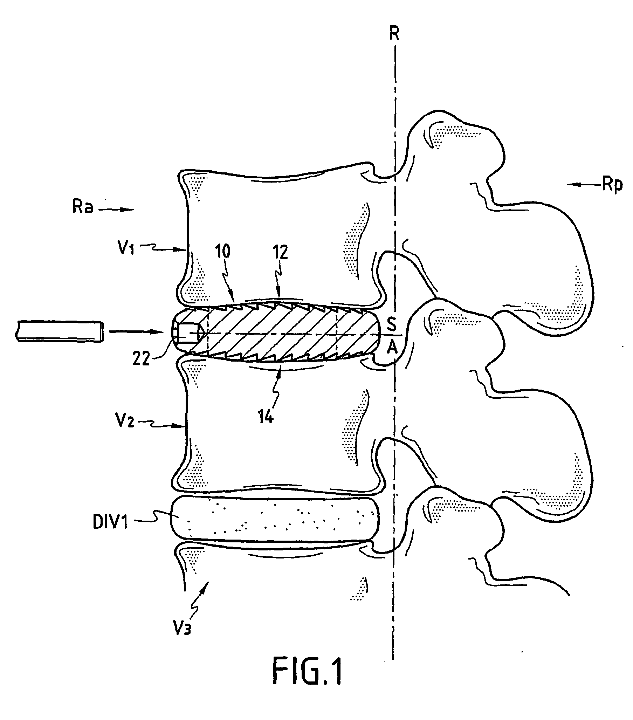

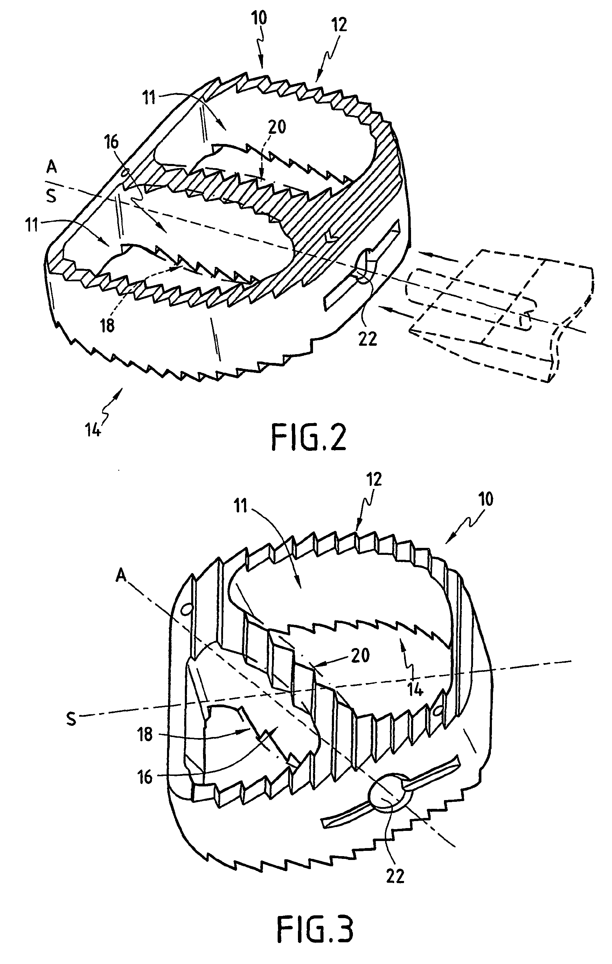

La Figure 1 illustre l'implant intersomatique inséré entre deux vertèbres contiguës. On se référera également à la Figure 2 pour décrire l'implant conforme à un premier mode de réalisation de l'invention et dans lequel il est, de façon préférentielle, inséré entre les vertèbres lombaires.

L'implant intersomatique présente une première partie 10 vue de profil sur la Figure 1. Le versant antérieur Ra du rachis fait face à la paroi avant du corps et le versant postérieur Rp du rachis fait face à la paroi arrière du corps.

L'implant intersomatique est destiné à immobiliser des greffons osseux entre deux vertèbres de façon à remplacer le disque intervertébral. Sur la Figure 1, le disque intervertébral DIV1 est représenté intact entre les vertèbre V2 et V3.

Le canal rachidien s'étendant le long du rachis selon l'axe R contient la moelle épinière et on comprend, en raison de la fragilité de cette dernière, que l'insertion de l'implant entre deux vertèbres se fait, préférentiellement, selon une voie d'abord antérieure. C'est notamment le cas dans la majorité des interventions pour les hernies discales cervicales.

Sur la Figure 2, où l'implant intervertébral est représenté de profil, on retrouve la première partie 10 de forme générale annulaire et définissant un évidement 11 divisé en deux par une seconde partie 16 de forme allongée selon un axe A, ladite seconde partie 16 reliant deux portions en regard de la première partie 10. Le premier bord supérieur 12 de la première partie 10 est sensiblement situé dans un plan parallèle au plan défini par le premier bord inférieur 14 de la première partie 10. Chacun de ces premiers bords, 12, 14, lorsque l'implant est inséré entre les vertèbres, est en contact avec le plateau vertébral qui lui correspond.

Les évidements 11 sont destinés à recevoir les greffons osseux qui sont en contact dans leur partie supérieure avec le plateau vertébral inférieur de la vertèbre supérieure et dans leur partie inférieure avec le plateau vertébral supérieur de la vertèbre inférieure. Ainsi, les greffons sont contenus et immobilisés entre les vertèbres de manière à permettre à la greffe de prendre et de solidariser l'ensemble. Ces greffons sont généralement des greffons corticaux prélevés sur la crête iliaque antérieure dont l'épaisseur correspond sensiblement à l'espace intersomatique. Il va de soi que, lorsque la liaison osseuse entre les vertèbres est établie, les implants intervertébraux restent à demeure et font partie intégrante de la liaison.

Afin que l'implant soit aussi stable que possible après son insertion entre les vertèbres, la seconde partie 16, qui relie les deux bords opposés de la première partie 10, présente des seconds bords inférieur 18 et supérieur 20 de forme curviligne faisant saillie en dehors de la première partie 10. Cette géométrie permet de respecter la forme biconcave de l'espace intersomatique et de constituer des points d'appui supplémentaires de l'implant contre les plateaux vertébraux dans leur partie centrale.

En préalable à l'insertion de l'implant muni de ses greffons osseux, le disque intervertébral endommagé est extrait de manière à permettre l'enfoncement de l'implant. Ensuite l'implant est enfoncé dans l'espace intervertébral selon une direction prédéterminée.

Cette direction prédéterminée dépend du niveau vertébral auquel l'intervention est réalisée et de l'indication thérapeutique.

L'implant intersomatique tel que représenté sur la Figure 2 est destiné à être inséré sur le versant antérieur du rachis. Il présente un trou borgne 22 percé dans la paroi de la première partie 10 de forme annulaire et se prolonge dans la seconde partie 16 selon son axe principal A, lequel est confondu avec l'axe de symétrie S de la première partie 10. L'extrémité de l'outil d'insertion est introduite dans le trou borgne 22 et prend appui dans ladite seconde partie 16 de renfort de façon à maintenir et à guider l'implant lors de son enfoncement dans l'espace intersomatique.

En effet, une caractéristique de l'invention réside dans l'insertion de la seconde partie 16 de forme allongée dans la première partie afin de permettre à cette dernière de résister aux contraintes qui lui sont appliquées lors de l'impaction. Pour que la résistance de l'implant à la déformation soit optimale les impacts doivent être appliqués selon l'axe A de la seconde partie 16. Or, le trou borgne 22 maintient l'outil d'impaction dans une direction constante par rapport à l'implant, selon l'axe A de ladite pièce. Par ailleurs, le fait que l'extrémité de l'outil d'insertion soit partiellement solidaire de l'implant permet de maîtriser la trajectoire de l'implant entre les corps vertébraux.

Le blocage de l'implant entre les vertèbres est fonction des forces de frottement s'exerçant à l'interface entre les bords 12, 14, 18 et 20 de la première partie 10 et de la seconde partie 16 et les plateaux vertébraux. Afin d'augmenter ces forces de frottement, les bords 12, 14, 18 et 20 présentent des cannelures formant des points d'ancrage dans les plateaux vertébraux.

Selon un mode particulier de mise en oeuvre, les cannelures sont inclinées dans le sens opposé à la direction d'insertion, de sorte que.dans le sens de l'insertion les forces de frottement de l'implant contre les vertèbres sont inférieures aux forces de frottement apparaissant dans le sens opposé. Ainsi, l'implant est il bloqué dans le sens opposé à sa direction d'insertion.

La première partie 10 de forme annulaire tel que représentée sur la Figure 2 présente un axe de symétrie S, lequel est confondu avec l'axe de symétrie longitudinal de la seconde partie 16, ce qui permet une insertion perpendiculaire au versant antérieur du rachis. Par ailleurs, la forme générale de ladite première partie 10 est sensiblement hémicirculaire de manière à correspondre sensiblement à la forme des plateaux vertébraux. Ainsi, la surface de greffe définie par l'implant est , elle optimale.

Selon un autre mode particulier de mise en oeuvre de l'invention, illustré par la Figure 3, la seconde partie 16 est oblique par rapport à l'axe de symétrie S de la première partie 10. De même, le trou borgne 22, perçant le bord latéral de la première partie 10 de forme annulaire, se prolonge dans ladite seconde partie 16 selon son axe A, oblique par rapport à l'axe de symétrie S.

Cette configuration permet une insertion de l'implant selon une direction oblique par rapport au versant antérieur du rachis, ce qui peut être nécessaire pour certains espaces intervertébraux difficilement accessibles.

Telle que représentée sur la Figure 3 la seconde partie allongée d'axe A et l'axe de symétrie S de la première partie forment un angle sensiblement égal à 45°. Cependant, la seconde partie peut être disposée de façon à former un angle compris entre 0 et 90°avec ledit axe de symétrie S.

Selon un mode préféré de mise en oeuvre l'implant est réalisé dans un matériau macromoléculaire présentant des propriétés de déformabilité élastique proches de celles de l'os et plus précisément comprises entre celles de l'os cortical et celles de l'os spongieux.

Le module d'élasticité ou module d'Young, est pour l'os spongieux de l'ordre de 0,1 GPa et pour l'os cortical de l'ordre de 12 GPa. Par conséquent, le matériau macromoléculaire est choisi parmi les matériaux synthétiques présentant un module d'élasticité compris entre 0,1 et 12 GPa.

De manière préférentielle le matériau présente un module d'élasticité compris entre 1 et 6 GPa de façon à constituer un bon compromis pour lequel, lorsqu'il est inséré entre deux vertèbres, il ne procure aucune discontinuité dans les propriétés mécaniques élastiques de l'ensemble formé par les vertèbres et l'implant.

Avantageusement, l'implant est moulé d'une seule pièce en polyéther-éther-cétone. Ce matériau présente un module d'élasticité de 3,5 GPa.

Le fait de pouvoir mouler l'implant est économiquement avantageux et permet de produire des cages intersomatiques à un coût inférieur aux coûts de production des cages en alliage de titane.

Selon encore un autre mode particulier de mise en oeuvre, la surface ouverte de l'implant correspondant à la somme des surfaces définies par l'intersection d'un plan moyen de l'implant et desdits deux logements, divisée par la surface totale définie par l'intersection du plan moyen avec les contours de ladite première partie correspond à un ratio compris entre 0,75 et 0,95.

On décrira maintenant un autre mode particulier de réalisation de l'invention, en référence aux Figures 3, 4 et 5, dans lequel l'implant est préférentiellement inséré entre les vertèbres cervicales.

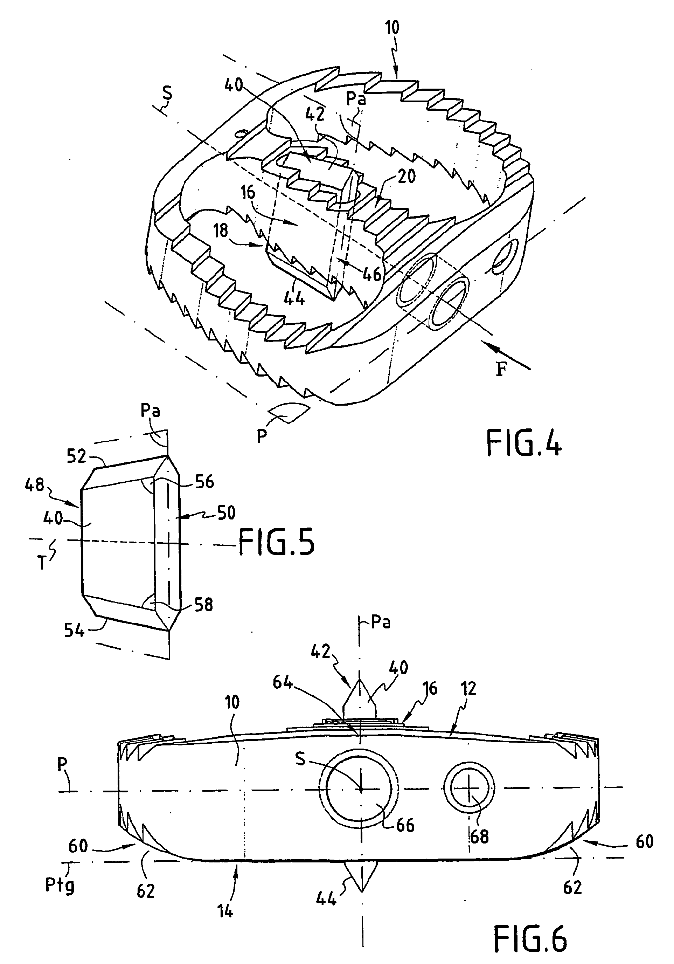

On retrouve sur la Figure 4 un implant, comportant une première partie 10 et une seconde partie 16 reliant deux bords opposés de la première partie 10. La seconde partie 16 présente, de manière analogue à la seconde partie représentée sur la Figure 2, un second bord inférieur 18 et un second bord supérieur 20. En revanche, elle est munie d'une pièce d'ancrage 40, qui est préférentiellement réalisée en alliage de titane, de façon à être bio-compatible et extrêmement résistante. La pièce d'ancrage 40 présente une première extrémité 42 qui débouche dans le second bord supérieur 20 et une seconde extrémité 44 qui débouche dans le second bord inférieur 18.

Pour ce faire, la seconde partie 16 présente un évidement 46 de forme générale parallélépipédique rectangle, qui débouche dans le second bord inférieur 18 et dans le second bord supérieur 20 et dont les quatre arrêtes formées par les parois internes de l'évidement 46 sont sensiblement perpendiculaires au plan moyen P de la première partie 10. De la sorte, la pièce d'ancrage 40 dont les dimensions sont légèrement supérieures à celles de l'évidement 46, est susceptible de traverser à force l'évidement 46 de part en part. Ainsi, elle est maintenue en position fixe par rapport à la seconde partie 16, par frottement contre les parois internes de l'évidement 46 qui s'écartent sensiblement les unes des autres.

Bien évidemment, les dimensions de la pièce d'ancrage 40, entre les deux extrémités 42 et 44 sont supérieures aux dimensions de l'évidement 46 de façon que les deux extrémités 42 et 44 fassent saillie dans chacun des second bords 18, 20. ,

La Figure 5 illustre la pièce d'ancrage 40 en perspective. Elle est de forme trapézoïdale et présente deux premiers bords opposés 48 et 50 parallèles entre eux formant les bases du trapèze et deux seconds bords opposés 52 et 54 formant un angle identique avec l'axe de symétrie T de la pièce d'ancrage 40. Ainsi, la pièce d'ancrage 40 présente deux coins 56 et 58 identiques à angle aigus susceptible de pénétrer dans la surface des plateaux vertébraux.

En outre, les deux seconds bords opposés 52 et 54 ont un profil aigu, pour pénétrer de manière encore plus aisée dans les plateaux vertébraux.

La pièce d'ancrage 40 définit un plan moyen Pa, et elle est insérée dans l'évidement 46, comme l'illustre la Figure 4, de façon que le plan moyen Pa soit perpendiculaire au plan moyen P de la première partie 10. En outre, elle est insérée de telle façon, que la direction F d'introduction de l'implant dans l'espace intervertébral corresponde à une direction allant du premiers bord 50, de plus grande dimension vers son bord opposé 48 de plus petite dimension. On comprend que cette disposition évite que la pièce d'ancrage ne freine l'introduction de l'implant dans l'espace intervertébral lorsque les extrémités 42 et 44 sont en appui contre les plateaux vertébraux des deux vertèbres. On comprend également que les deux coins 56 et 58 de la pièce d'ancrage qui s'incrustent dans les plateaux vertébraux lors de l'introduction de l'implant constituent des moyens de blocage important de la pièce d'ancrage 40 et donc de l'implant, notamment dans la direction opposée à la direction d'introduction. Ainsi, l'implant est maintenu de façon plus efficace entre les deux vertèbres.

La Figure 6 montre l'implant vue de face, muni de la pièce d'ancrage 40 dont la première extrémité 42 apparaît au-dessus de la première partie 10 et la seconde extrémité 44 en dessous.

Dans ce mode particulier de réalisation, où l'implant est généralement introduit entre des vertèbres cervicales, la première partie 10 n'est pas strictement symétrique par rapport à son plan moyen P. En effet, comme l'illustre la Figure 6, le premier bord inférieur 14 présente des portions incurvées 60 sur les parties latérales de la première partie 10, faisant apparaître des espaces libres 62 entre le plan tangent Ptg de la première partie 10 et le premier bord inférieur 14. De plus, le premier bord supérieur de la première partie présente une portion convexe 64 où la seconde partie 16 relie la première partie 10.

Grâce à cette configuration, l'implant intersomatique coïncide parfaitement avec la géométrie de l'espace intervertébral, notamment au niveau des vertèbres cervicales.

Compte tenu de cette dissymétrie, l'implant doit être introduit dans un sens prédéterminé. Et pour ce faire, un trou borgne principal 66 situé suivant l'axe de symétrie S est susceptible de recevoir l'outil et un trou d'orientation 68 permet d'orienter l'implant dans le sens prédéterminé.

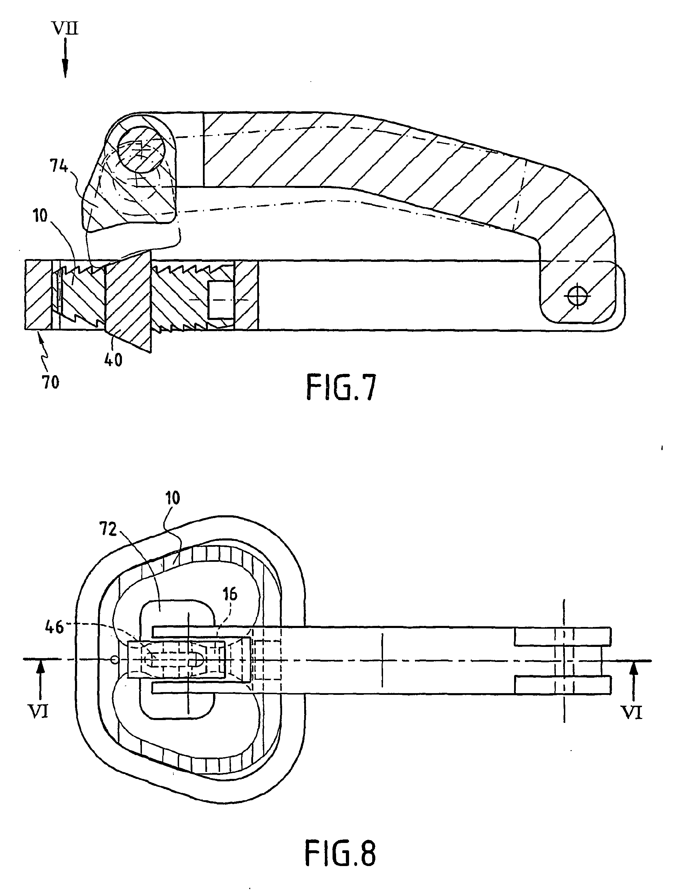

Selon un autre objet l'invention concerne une pince à implant intersomatique destinée à insérer ou à désinsérer ladite pièce d'ancrage 40 dans ledit évidement 46. On décrira la pince en référence aux Figures 7 et 8.

La pince à implant comporte une première mâchoire 70 susceptible de recevoir l'implant qui est constitué de la première partie 10 dans laquelle la seconde partie 16 relie deux bords opposés, la pièce d'ancrage 40 étant insérée dans cette seconde partie 16. La première mâchoire 70 présente un évidement central 72 illustré sur la Figure 8, permettant le passage de la pièce d'ancrage 40 lorsqu'elle est forcée à travers l'évidement 46 de la seconde partie 16. La pince comporte une seconde mâchoire 74 destinée à prendre appui contre la pièce d'ancrage 40, afin de l'insérer lorsque les mâchoires 70 et 74 sont rapprochées l'une de l'autre. Pour ce faire, chaque mâchoire 70, 74 est prolongée par un manche, les manches étant reliés ensemble de façon à pouvoir pivoter l'un par rapport à l'autre.

En outre, lorsque la pièce d'ancrage est préinstallée dans la seconde partie 16, il est aisé de l'extraire à force au moyen de la pince si l'intervention ne requiert pas l'introduction d'un implant équipé d'une pièce d'ancrage.

## Revendications

1. Implant intersomatique apte à contenir au moins un greffon osseux et à être inséré dans l'espace intervertébral selon une direction prédéterminée, qu'il comprend:
- une première partie (10), de forme générale annulaire, entourant un évidement (11) et présentant un premier bord supérieur (12) et un premier bord inférieur (14), et,
- une seconde partie (16) de forme allongée selon son axe principal (A) présentant un second bord inférieur (18) et un second bord supérieur (20), et qui relie selon ladite direction prédéterminée deux portions opposées en regard de ladite première partie (10) en divisant ledit évidement (11) en deux logements aptes à contenir ledit greffon osseux, **caractérisé en ce qu'**au moins une portion desdits seconds bords inférieur et supérieur font saillie en dehors de l'espace défini par lesdits premiers bords inférieur (14) et supérieur (12) de ladite première partie (10) et sont de forme curviligne et leurs extrémités rejoignent respectivement lesdits premiers bords inférieur (14) et supérieur (12) de ladite première partie (10) de telle manière que les deuxièmes bords respectent la forme biconcave de l'espace intersomatique et constituent des points d'appui supplémentaires de l'implant contre les plateaux vertébraux dans leur partie centrale.

2. Implant intersomatique selon la revendication 1, **caractérisé en ce que** ladite première partie (10) et ladite seconde partie (16) sont reliées ensemble de façon à former une seule et même pièce.

3. Implant intersomatique selon l'une quelconque des revendications 1 et 2, **caractérisé en ce que** ladite première partie (10) est percée latéralement selon ledit axe principal (A) de ladite seconde partie de façon à ménager un trou borgne (22) se prolongeant dans ladite seconde partie (16), ledit trou borgne (22) étant apte à recevoir un outil d'insertion.

4. Implant intersomatique selon l'une quelconque des revendications 1 à 3, **caractérisé en ce que** lesdits bords inférieurs et supérieurs (12, 14, 18, 20) desdites première et seconde parties présentent des cannelures aptes à constituer des points d'ancrage dans lesdites vertèbres.

5. Implant intersomatique selon l'une quelconque des revendications 1 à 4, **caractérisé en ce que** ladite première partie (10) de forme générale annulaire présente au moins un axe de symétrie (S).

6. Implant intersomatique selon la revendication 5, **caractérisé en ce que** ladite seconde partie (16) relie lesdites deux portions opposées de ladite première partie (10) selon ledit axe de symétrie (S).

7. Implant intersomatique selon la revendication 5, **caractérisé en ce que** ladite seconde partie (16) relie lesdites deux portions opposées de ladite première partie (10) selon une direction faisant un angle compris entre 0 et 90° avec ledit axe de symétrie (S).

8. Implant intersomatique selon l'une quelconque des revendications 5 à 7, **caractérisé en ce que** ladite première partie (10) présente une forme générale sensiblement hémicirculaire

9. Implant intersomatique selon l'une quelconque des revendications 1 à 8, **caractérisé en ce que** ladite première partie (10) et ladite seconde partie (16) sont moulées d'une seule pièce dans un matériau macromoléculaire.

10. Implant intersomatique selon l'une quelconque des revendications 1 à 9, **caractérisé en ce que** ladite première partie (10) et ladite seconde partie (16) sont en polyéther-éther-cétone.

11. Implant intersomatique selon l'une quelconque des revendications 1 à 10, **caractérisé en ce que** ladite seconde partie comprend en outre des moyens d'ancrage débouchant respectivement dans ledit second bord supérieur et dans ledit second bord inférieur.

12. Implant intersomatique selon la revendication 11, **caractérisé en ce que** lesdits moyens d'ancrage sont constitués d'une pièce d'ancrage présentant deux extrémités et **en ce que** ladite seconde partie présente un évidement débouchant dans lesdits seconds bord inférieur et supérieur de façon que ladite pièce d'ancrage, insérée dans ledit évidement, traverse ladite seconde partie de part en part, la première extrémité débouchant dans ledit second bord supérieur et la seconde extrémité débouchant dans ledit second bord inférieur de ladite seconde partie.

13. Implant intersomatique selon la revendication 12, **caractérisé en ce que** ladite pièce d'ancrage est sensiblement de forme trapézoïdale, ses deux extrémités étant définies par les deux côtés non consécutifs et non parallèles entre eux.

14. Implant intersomatique selon la revendication 13, **caractérisé en ce que** ladite pièce d'ancrage présente un plan moyen, et **en ce qu'**elle est insérée dans ladite seconde partie de façon que ledit axe principal A de ladite seconde partie coupe les deux bases du trapèze et que son plan moyen soit sensiblement perpendiculaire au plan moyen de ladite première partie.

15. Implant intersomatique selon la revendication 13 ou 14, **caractérisé en ce que** l'une des bases du trapèze forme deux angles aigus avec les deux autres côtés non parallèles entre eux, et **en ce que** ladite pièce d'ancrage est insérée dans ladite seconde partie de façon que la direction d'insertion dudit implant s'effectue de la plus grande base vers la plus petite base du trapèze.

16. Implant selon l'une quelconque des revendications 12 à 15, **caractérisé en ce que** ladite pièce d'ancrage est réalisée en alliage de titane.

## Claims

1. An intersomatic implant suitable for containing at least one bone graft and for being inserted in the intervertebral space along a predetermined direction, the implant comprising:
- a first portion (10) of generally annular shape surrounding an aperture (11) and presenting a top first edge (12) and a bottom first edge (14); and
- a second portion (16) of elongate shape along its main axis (A) presenting a bottom second edge (18) and a top second edge (20), and interconnecting two facing opposite regions of said first portion (10) along said predetermined direction, thereby dividing said aperture (11) into two housings suitable for containing said bone graft, the implant being **characterised in that** at least one portion of said bottom and top second edges projects out from the space defined by said bottom and top first edges (14, 12) of said first portion (10) and said bottom and top second edges are curvilinear in shape with their ends running into said bottom and top first edges (14, 12) respectively of said first portion (10) in such a manner that the second edges comply with the biconcave shape of the intersomatic space and serve to establish additional bearing points for the implant against the central portions of the vertebral plates.

2. An intersomatic implant according to claim 1, **characterised in that** said first portion (10) and said second portion (16) are connected together so as to form a single piece.

3. An intersomatic implant according to claim 1 or claim 2, **characterised in that** said first portion (10) is pierced laterally along said main axis (A) of said second portion so as to provide a blind hole (22) extending in said second portion (16), said blind hole (22) being suitable for receiving an insertion tool.

4. An intersomatic implant according to any one of claims 1 to 3, **characterised in that** said bottom and top edges (12, 14, 18, 20) of said first and second portions present serrations suitable for constituting anchoring points in said vertebrae.

5. An intersomatic implant according to any one of claims 1 to 4, **characterised in that** said first portion (10) of generally annular shape has at least one axis of symmetry (S).

6. An intersomatic implant according to claim 5, **characterised in that** said second portion (16) interconnects said two opposite regions of said portion (10) along said axis of symmetry (S).

7. An intersomatic implant according to claim 5, **characterised in that** said second portion (16) interconnects said two opposite regions of said first portion (10) along a direction that is at an angle lying in the range 0° to 90° with said axis of symmetry (S).

8. An intersomatic implant according to any one of claims 5 to 7, **characterised in that** said first portion (10) is generally substantially semicircular in shape.

9. An intersomatic implant according to any one of claims 1 to 8, **characterised in that** said first portion (10) and said second portion (16) are moulded as a single piece of macromolecular material.

10. An intersomatic implant according to any one of claims 1 to 9, **characterised in that** said first portion (10) and said second portion (16) are made of polyether ether ketone.

11. An intersomatic implant according to any one of claims 1 to 11, **characterised in that** said second portion further comprises anchoring means opening out into said top and bottom second edges, respectively.

12. An intersomatic implant according to claim 11, **characterised in that** said anchoring means are constituted by an anchoring piece having two ends, and **in that** said second portion has a slot opening out into said bottom and top second edges so that said anchoring piece when inserted in said slot passes right through said second portion, the first end opening out into said top second edge and the second end opening out into said bottom second edge of said second portion.

13. An intersomatic implant according to claim 12, **characterised in that** said anchoring piece is substantially trapezoidal in shape, its two ends being defined by the two non-consecutive and non-parallel sides thereof.

14. An intersomatic implant according to claim 13, **characterised in that** said anchoring piece presents a midplane, and **in that** it is inserted in said second portion so that said main axis A of said second portion intersects the two bases of the trapezoid, and its midplane is substantially perpendicular to the midplane of said first portion.

15. An intersomatic implant according to claim 13 or claim 14, **characterised in that** one of the bases of the trapezoid forms two acute angles with the two other non-parallel sides, and **in that** said anchoring piece is inserted in said second portion so that the insertion direction of said implant extends from the longer base towards the shorter base of the trapezoid.

16. An implant according to any one of claims 12 to 15, **characterised in that** said anchoring piece is made of titanium alloy.

## Patentansprüche

1. Intervertebrales Implantat, das geeignet ist, wenigstens einen Knochenspan zu enthalten und in einer vorbestimmten Richtung in den intervertebralen Zwischenraum eingeführt zu werden, welches umfasst:
- einen ersten, allgemein ringförmigen Teil (10), der eine Aussparung (11) umgibt und einen ersten oberen Rand (12) und einen ersten unteren Rand (14) aufweist, und
- einen zweiten, entlang seiner Hauptachse (A) langgezogenen Teil (16), der einen zweiten unteren Rand (18) und einen zweiten oberen Rand (20) aufweist und in der vorbestimmten Richtung zwei gegenüberliegende Abschnitte des ersten Teils (10) miteinander verbindet und hierbei die Aussparung (11) in zwei Aufnahmen unterteilt, die den Knochenspan zu enthalten vermögen,
**dadurch gekennzeichnet, dass** wenigstens ein Abschnitt des zweiten unteren und oberen Rands aus dem durch den ersten unteren (14) und oberen (12) Rand des ersten Teils (10) gebildeten Raum übersteht und eine gekrümmte Form hat, und dessen Enden den ersten unteren (14) bzw. oberen (12) Rand des ersten Teils (10) derart verbinden, dass die zweiten Ränder die bikonkave Form des intervertebralen Zwischenraums beachten und zusätzliche Auflagepunkte des Implantats an den Wirbelplatten in deren Mittelteil bilden.

2. Intervertebrales Implantat nach Anspruch 1,
**dadurch gekennzeichnet, dass** der erste Teil (10) und der zweite Teil (16) derart miteinander verbunden sind, dass sie ein einziges Stück bilden.

3. Intervertebrales Implantat nach einem der Ansprüche 1 oder 2,
**dadurch gekennzeichnet, dass** der erste Teil (10) entlang der Hauptachse (A) des zweiten Teils seitlich derart durchbohrt ist, dass ein Sackloch (22) ausgebildet ist, das sich in dem zweiten Teil (16) fortsetzt, wobei das Sackloch (22) ein Werkzeug zum Einführen aufzunehmen vermag.

4. Intervertebrales Implantat nach einem der Ansprüche 1 bis 3,
**dadurch gekennzeichnet, dass** die unteren und oberen Ränder (12, 14, 18, 20) des ersten und des zweiten Teils Kerben aufweisen, die zum Bilden von Verankerungspunkten in den Wirbel geeignet sind.

5. Intervertebrales Implantat nach einem der Ansprüche 1 bis 4,
**dadurch gekennzeichnet, dass** der erste, allgemein ringförmige Teil (10) wenigstens eine Symmetrieachse (S) aufweist.

6. Intervertebrales Implantat nach Anspruch 5,
**dadurch gekennzeichnet, dass** der zweite Teil (16) die beiden gegenüberliegenden Abschnitte des ersten Teils (10) entlang der Symmetrieachse (S) verbindet.

7. Intervertebrales Implantat nach Anspruch 5,
**dadurch gekennzeichnet, dass** der zweite Teil (16) die beiden gegenüberliegenden Abschnitte des ersten Teils (10) in einer Richtung verbindet, die mit der Symmetrieachse (S) einen Winkel zwischen 0° und 90° bildet.

8. Intervertebrales Implantat nach einem der Ansprüche 5 bis 7,
**dadurch gekennzeichnet, dass** der erste Teil (10) im Wesentlichen die allgemeine Form eines Halbkreises aufweist.

9. Intervertebrales Implantat nach einem der Ansprüche 1 bis 8,
**dadurch gekennzeichnet, dass** der erste Teil (10) und der zweite Teil (16) aus einem makromolekularen Werkstoff einstückig geformt sind.

10. Intervertebrales Implantat nach einem der Ansprüche 1 bis 9,
**dadurch gekennzeichnet, dass** der erste Teil (10) und der zweite Teil (16) aus Polyetheretherketon sind.

11. Intervertebrales Implantat nach einem der Ansprüche 1 bis 10,
**dadurch gekennzeichnet, dass** der zweite Teil ferner Verankerungsmittel umfasst, die jeweils in den zweiten oberen Rand und in den zweiten unteren Rand münden.

12. Intervertebrales Implantat nach Anspruch 11,
**dadurch gekennzeichnet, dass** die Verankerungsmittel aus einem Verankerungsstück mit zwei Enden gebildet sind, und dass der zweite Teil eine Aussparung aufweist, die derart in den zweiten unteren und oberen Rand mündet, dass das in die Aussparung eingeführte Verankerungsstück den zweiten Teil vollständig durchquert, wobei das erste Ende in den zweiten oberen Rand mündet und das zweite Ende in den zweiten unteren Rand des zweiten Teils mündet.

13. Intervertebrales Implantat nach Anspruch 12,
**dadurch gekennzeichnet, dass** das Verankerungsstück im Wesentlichen trapezförmig ist, wobei seine beiden Enden durch die beiden nicht aufeinanderfolgenden und zueinander nicht parallelen Seiten gebildet sind.

14. Intervertebrales Implantat nach Anspruch 13,
**dadurch gekennzeichnet, dass** das Verankerungsstück eine Mittelebene aufweist, und dass es in den zweiten Teil derart eingeführt ist, dass die Hauptachse (A) des zweiten Teils die beiden Grundlinien des Trapezes schneidet, und dass seine Mittelebene im Wesentlichen lotrecht zur Mittelebene des ersten Teils ist.

15. Intervertebrales Implantat nach einem der Ansprüche 13 oder 14,
**dadurch gekennzeichnet, dass** eine der Grundlinien des Trapezes mit den beiden anderen zueinander nicht parallelen Seiten zwei spitze Winkel bildet, und dass das Verankerungsstück in den zweiten Teil derart eingeführt ist, dass das Implantat von der größten Grundlinie aus in Richtung der kleinsten Grundlinie des Trapezes eingeführt wird.

16. Intervertebrales Implantat nach einem der Ansprüche 12 bis 15,
**dadurch gekennzeichnet, dass** das Verankerungsstück aus Titanlegierung ausgeführt ist.
